# EUROPÄISCHE PATENTSCHRIFT

(11) **EP 0 991 364 B1**
(45) Veröffentlichungstag und Bekanntmachung des Hinweises auf die Patenterteilung: **17.01.2007**
(21) Anmeldenummer: 99916919.6
(22) Anmeldetag: 14.04.1999
(51) Int. Cl.: A61B 18/14

(54) **RADIOFREQUENZ ABLATIONSANORDNUNG**
RF ABLATION SYSTEM
DISPOSITIF D'ABLATION DE HAUTE FREQUENCE

(30) Priorität: 15.04.1998 DE 19817553
(43) Veröffentlichungstag der Anmeldung: 12.04.2000
(73) Patentinhaber: BIOTRONIK GmbH & Co. KG, 12359 Berlin (DE)
(72) Erfinder: TÖLLNER, Thomas, D-12047 Berlin (DE)
(74) Vertreter: Lindner-Vogt, Karin L.
(86) Internationale Anmeldenummer: PCT/EP1999/002515
(87) Internationale Veröffentlichungsnummer: WO 1999/052461

(56) Entgegenhaltungen:
- WO-A-96/00039
- US-A- 5 722 401

## Beschreibung

Die Erfindung betrifft eine Ablationsanordnung, die einen Ablationskatheter mit einer Mehrzahl von Ablationselektroden und eine Neutralelektrode sowie mindestens eine Energiequelle und Schaltmittel zum Verbinden der Elektroden mit der Energiequelle umfaßt. Außerdem betrifft die Erfindung ein Verfahren zum Ansteuern der Elektroden einer solchen Ablationsanordnung.

Der Begriff Ablationselektrode steht dabei für jedes kleinflächige Energieauskoppelelement, während Neutralelektrode für jedes vergleichsweise großflächige Energieauskoppelelement steht. Weiterhin wird unter einem Ablationskatheter jede Anordnung von kleinflächigen Energieauskoppelelementen verstanden, die durch entsprechende Verbindungsmittel räumlich einander zugeordnet sind.

Die Erfindung betrifft insbesondere das allgemeine Problem des Erzeugens linienförmiger Läsionen mit durchgängig ausreichender Tiefe, um beispielsweise eine anhaltend kurative Wirkung bei Ablation von Tachyarhythmien zu erzielen.

Zum Erzeugen derartiger linearer Läsionen ist es beispielsweise bekannt, einen Ablationskatheter mit einer relativ langgestreckten Elektrode am freien Ende des Katheters zu verwenden. Die lineare Läsion wird dadurch hervorgerufen, daß die Elektrode in ihrer Längsrichtung langsam bewegt wird, während sie mit einer Energiequelle verbunden ist.

Eine andere Ablationsanordnungen ist aus der internationalen Patentanmeldung WO-A-97/20510 bekannt. Dort ist ein HF-Ablationssystem beschrieben, mit dem simultan und in vorgegebener Phasenzuordnung über mehrere Elektroden eines multipolaren Katheters HF-Energie abgegeben werden kann. Hierbei wird eine der Elektrodenzahl entsprechende Anzahl von Leistungs-Modulen eingesetzt. Diese Anordnung ist konstruktiv aufwendig und teuer. Außerdem sind die verschiedenen Elektrodensysteme nicht gegeneinander entkoppelt, so daß sich darauf basierende Meß- oder Regelkreise gegenseitig beeinflussen.

Vorrichtungen und Verfahren gemäß Oberbegriff der Ansprüche 1 und 7 sind aus US-A-5 722 401 bekannt.

Der Erfindung liegt deshalb die Aufgabe zugrunde, Mittel und Wege anzugeben, mit denen insbesondere lineare Ablationen ohne großen Aufwand zu erzeugen sind.

Diese Aufgabe wird erfindungsgemäß durch eine Ablationsanordnung der eingangs genannten Art gelöst, die mit den Schaltelementen verbundene Steuermittel umfaßt, die derart gestaltet sind, daß sowohl von zwei Ablationselektroden als auch von einer Ablationselektrode zusammen mit der Neutral-Elektrode gebildete Elektrodenpaare mit der Energiequelle in einer vorgebbaren Reihenfolge verbindbar sind.

Erfindungsgemäß wird die Aufgabe ebenso durch ein Verfahren der eingangs genannten Art gelöst, bei dem Elektrodenpaare mit der Energiequelle in einer vorgegebenen Reihenfolge verbunden werden, wobei die Elektrodenpaare sowohl von zwei Ablationselektroden als auch von einer Ablationselektrode zusammen mit der Neutral-Elektrode gebildet werden.

Die Erfindung schließt die Erkenntnis ein, daß sich unterschiedliche Läsionsmuster ausbilden, je nach dem, ob zwei Ablationselektroden mit der Energiequelle verbunden werden, oder ob eine Ablationselektrode und die Neutral-Elektrode mit der Energiequelle verbunden werden. Eine Erklärung dieses Phänomens, auf der die Erfindung beruht, besteht darin, daß sich zwischen den entsprechenden Elektroden eines Elektrodenpaares ein elektrisches Feld ausbildet, das im Bereich der kleinflächigen Ablationselektroden stark gebündelt ist und eine hohe Energiedichte aufweist, während die Neutral-Elektrode so großflächig ist, daß die Energiedichte des Feldes ausreichend gering ist, um keine Läsionen hervorzurufen. Im Sinne der Erfindung führt dies dazu, daß die Läsionen beim Ansteuern eines aus zwei Ablationselektroden bestehenden Elektrodenpaares zwischen diesen Ablationselektroden auftreten, während die Läsionen beim Ansteuern eines aus einer Ablationselektrode und der Neutralelektrode bestehenden Elektrodenpaares in der Nachbarschaft der einen Ablationselektrode auftreten. Die Läsionen können sich z. B. hinsichtlich ihrer Tiefe unterscheiden: Aufgrund der elektrischen Eigenschaften des zu behandelnden Myokards im Verhältnis zum umgebenden Blut kommt es zu einer Konzentration des elektrischen Feldes zwischen zwei mit der Energiequelle verbundenen Ablationselektroden, und je nach Energiemenge, zu einer weniger tiefreichenden Läsion, während die von einer Ablationselektrode in Verbindung mit der Neutralelektrode erzielten Läsionen tendenziell tiefer ins Myokard reichen. Das Ansteuern eines Elektrodenpaares, bestehend aus zwei Ablationselektroden wird im folgenden bipolarer Betrieb genannt, während das Ansteuern eines Elektrodenpaares, bestehend aus einer Ablationselektrode und der Neutralelektrode als unipolarer Betrieb bezeichnet wird. Kern der Erfindung ist es, einen Ablationskatheter mit mehreren Ablationselektroden sowohl bipolar als auch unipolar zu betreiben, um auf diese Weise insbesondere lineare Ablationen zu erzeugen. Dementsprechend schließt die Reihenfolge, in der die Ablationselektroden betrieben werden, wie beansprucht, den unipolaren und den bipolaren Betrieb der Ablationselektroden ein.

Die Ablationsanordnung ist erfindungsgemäß so ausgebildet, daß die Ablationselektroden mit Abstand aufeinander folgend angeordnet sind und die Steuermittel derart gestaltet sind, daß die von zwei Ablationselektroden gebildeten Elektrodenpaare voneinander benachbarten Ablationselektroden gebildet sind. Bei der erfindungsgemäßen Ablationsanordnung sind die Steuermittel derart gestaltet, daß streng abwechselnd ein von Ablationselektroden gebildetes Elektrodenpaar und ein von einer der Ablationselektroden zusammen mit der Neutralelektrode gebildetes Elektrodenpaar mit der Energiequelle verbindbar sind. Das heißt, daß für den bipolaren Betrieb jeweils benachbarte Ablationselektroden herangezogen werden, und daß sich unipolarer und bipolarer Betrieb vorzugsweise ständig abwechseln.

Entsprechend zeichnet sich dies erfindungsgemäß Verfahren dadurch aus, daß die von den Ablationselektroden gebildeten Elektrodenpaare von einander benachbarten Ablationselektroden einer Mehrzahl von mit Abstand aufeinander folgenden Ablationselektroden gebildet werden. Bei dem erfindungsgemäß Verfahren werden streng abwechselnd ein von zwei Ablationselektroden gebildetes Elektrodenpaar und ein von einer der Ablationselektroden zusammen mit der Neutralelektrode gebildetes Elektrodenpaar mit der Energiequelle verbunden. Auch dies läuft auf abwechselnden unipolaren und bipolaren Betrieb hinaus, wobei für den bipolaren Betrieb vorzugsweise einander benachbarte Ablationselektroden mit der Energiequelle verbunden werden.

Bevorzugt ist auch ein Verfahren, bei dem zunächst ein Elektrodenpaar mit der Energiequelle verbunden wird, das von einer ersten Ablationselektrode aus einer Reihe von mit Abstand aufeinander folgenden Ablationselektroden zusammen mit der Neutralelektrode gebildet wird, anschließend ein von der ersten Ablationselektrode und einer auf diese folgenden zweiten Ablationselektrode gebildetes Elektrodenpaar, daraufhin ein von der zweiten Ablationselektrode und der Neutralelektrode gebildetes Elektrodenpaar, daraufhin wieder ein von der zweiten Ablationselektrode und der nächst folgenden Ablationselektrode gebildetes Elektrodenpaar, und bei dem in dieser abwechselnden Reihe fortgefahren wird, bis eine gewünschte Ablationslänge erzielt ist. Auf einen multipolaren Katheter mit einer Tip-Elektrode an seinem freien Ende und mehreren auf die Tip-Elektrode folgenden Ringelektroden übertragen, verläuft das Verfahren so, daßdie Energie der Energiequelle zunächst unipolar zwischen der Tip-Elektrode und der Neutralelektrode abgegeben wird, dann bipolar zwischen der Tip-Elektrode und der ersten Ringelektrode, dann wieder unipolar zwischen der ersten Ringelektrode und der Neutralelektrode, anschließend bipolar zwischen erster und zweiter Ringelektrode usw., bis der Abstand zwischen der ersten mit der Energiequelle verbundenen Elektrode und der letzten mit der Energiequelle verbundenen Elektrode einer gewünschten Länge entspricht. Die Ringelektroden sind nur beispielhaft genannt. Anstelle von Ringelektroden können auch Wendeln oder Arrays von Elektroden entsprechend angesteuert werden.

Eine weitere bevorzugte Verfahrensvariante zeichnet sich dadurch aus, daß eine oder mehrere der Ablationselektroden in der Reihenfolge der mit der Energiequelle zu verbindenden Elektroden ausgelassen werden, um Ablationslücken zu lassen. Mit dieser Verfahrensvariante wird nicht eine durchgehende lineare Ablation erzeugt, sondern eine abschnittsweise unterbrochene Ablation.

Bevorzugt ist auch ein Verfahren, bei dem die Leistung, mit der die von der Energiequelle stammende Energie über die Elektroden abgegeben wird, eine andere ist, wenn ein Elektrodenpaar von zwei Ablationselektroden gebildet wird, als wenn ein Elektrodenpaar von einer Ablationselektrode zusammen mit der neutralelektrode gebildet wird. Durch Differenzierung der Leistung für den unipolaren und den bipolaren Betrieb ist es möglich, die Tiefe der per Ablation erzeugten Läsionen im Myokard für die beiden Betriebsarten aneinander anzupassen, um ein optimales Ablationsergebnis zu erzeugen.

Allgemein, aber insbesondere auch in Verbindung mit der letztgenannten Verfahrensvariante wird ein Verfahren bevorzugt, bei dem die Leistung, mit der die von der Energiequelle stammende Energie für zwei Ablationselektroden abgegeben wird, derart durch Messen der Elektrodentemperatur geregelt wird, das für die Regelung zwei Temperaturwerte berücksichtigt werden, die jeweils der einen und der anderen Ablationselektrode zugeordnet sind. Die Berücksichtigung der beiden Temperaturwerte für die beiden ein Elektrodenpaar bildenden Ablationselektroden kann beispielsweise durch Interpolation geschehen. Die Temperaturwerte können aber auch auf andere Weise in die Leistungsregelung eingehen.

Die genannte Ablationsanordnung sowie das Verfahren bieten zum einen den Vorteil, daß sich mit ihnen gleichmäßige lineare Läsionen erzeugen lassen. Zum anderen kann der Abstand der Ablationselektroden voneinander an dem verwendeten Ablationskatheter im Vergleich zu herkömmlichen Ablationskathetern größer dimensioniert werden. Daraus resultiert eine erhöhte Flexibilität des Katheters und somit eine feinere Steuerung bei der Positionierung.

Die weitere Gestaltung der Ablationsanordnung kann derjenigen entsprechen, die in der deutschen Patentanmeldung 198 17 553.1 beschrieben ist.

Die Erfindung soll nun anhand eines Ausführungsbeispieles mit Hilfe der Figuren näher erläutert werden. Diese zeigen:
- - Figur 1: eine Prinzipdarstellung einer Ablationsanordnung;
- - Figur 2: ein Impulsdiagramm für die Schaltzustände von Schaltelementen, die zwischen den Elektroden und der Energiequelle angeordnet sind; und
- - Figur 3: ein Blockschaltbild einer Ausführungsvariante der Ablationsanordnung.

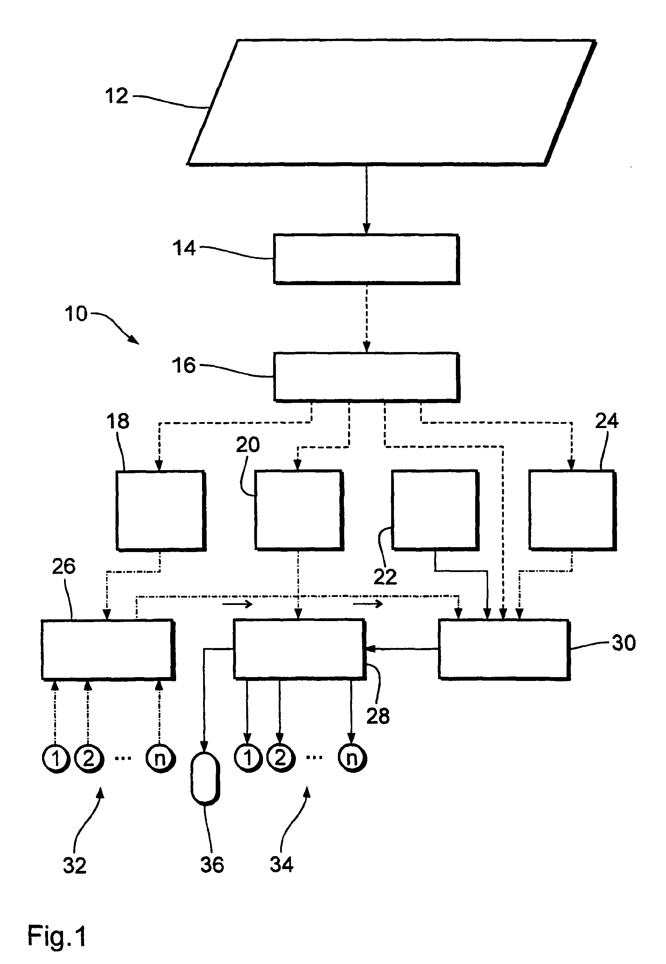

Fig. 1 zeigt eine Ablationsanordnung 10 in prinzipieller Darstellung, in der die einzelnen Bestandteile der Ablationsanordnung in Blöcken dargestellt sind und die Verbindungsleitungen zwischen den Bestandteilen durch gestrichelte, strichpunktierte und durchgezogene Linien. Die gestrichelten Linien bezeichnen dabei digitale Signalleitungen, die strichpunktierten Linien analoge Signalleitungen und die durchgezogenen Linien Leitungen für elektrische Leistungen. Die Pfeile an den Linien geben dabei die Flußrichtung der Information bzw. der elektrischen Leistungen an.

Die Funktionseinheiten der Ablationsanordnung 10 sind eine Eingabeeinheit 12, eine Speichereinheit 14, eine Steuereinheit 16, ein erster und ein zweiter Taktgeber 18 und 20, eine Energiequelle 22, ein dritter Taktgeber 24, ein erster und ein zweiter Multiplexer 26 und 28, eine Regeleinheit 30, sowie Temperatursensoren 32, Ablationselektroden 34 und schließlich eine Flächenelektrode 36 als Neutralelektrode.

Insbesondere für den Multiplexer 28, aber auch für den Multiplexer 26 kann jeweils ein mehrkanaliger Leistungsschalter eingesetzt werden, der von der Steuereinheit 16 gesteuert wird. Außerdem können die beiden Multiplexer oder Leistungsschalter zu einer Einheit zusammengefaßt sein.

Die Eingabeeinheit 12 dient der Parametereingabe und ist mit der Speichereinheit 14 verbunden. Einzugebende Parameter sind beispielsweise die Anzahl der Ablationselektroden 34, die Umschaltfrequenzen der Multiplexer 26 und 28, die Impulsdauer für eine Einzelablation, die Ablationsleistung für den unipolaren und den bipolaren Betrieb oder das Verhältnis dieser Leistungen bei einer Temperaturregelung, die maximale Ablationstemperatur sowie die gesamte Ablationszeit oder die Anzahl von Ablationssequenzen. Die eingegebenen Parameter werden in der Speichereinheit 14 gespeichert.

An die Speichereinheit 14 ist die Steuereinheit 16 angeschlossen, und hat über eine die Speichereinheit 14 mit der Steuerungseinheit 16 verbindende digitale Signalleitung Zugriff auf die gespeicherten Parameter. Die Steuereinheit 16 ist ausgangsseitig über digitale Signalleitungen an den ersten, zweiten und dritten Taktgenerator 18, 20 und 24 sowie an die Regeleinheit 30 angeschlossen.

Der erste Taktgeber 18 ist ausgangsseitig über eine analoge Signalleitung mit dem ersten Multiplexer 26 verbunden. An den ersten Multiplexer sind über analoge Signalleitungen eine Mehrzahl von Temperatursensoren 32 angeschlossen. Die Temperatursensoren 32 sind beispielsweise Thermoelemente. Außerdem ist der erste Multiplexer 26 an seinem Ausgang über eine analoge Signalleitung mit der Regeleinheit 30 verbunden. Über die analogen Signalleitungen gibt der erste Multiplexer 26 jeweils einen der von den Temperatursensoren 32 ermittelten Temperaturwerte an die Regeleinheit 30 weiter. Es hängt dabei vom Schaltzustand des ersten Multiplexers 26 ab, von welchem der Temperatursensoren der an die Regeleinheit 30 weitergeleitete Temperaturwert stammt.

Derzweite Multiplexer 28 ist eingangsseitig über eine analoge Signalleitung mit dem zweiten Taktgeber 20 verbunden. Außerdem ist der zweite Multiplexer 28 über eine elektrische Leitung mit der Regeleinheit 30 verbunden. Ausgangsseitig ist der zweite Multiplexer 28 über jeweils eine elektrische Leitung mit jeweils einer der Ablationselektroden 34 verbunden sowie mit der Flächen- oder Neutralelektrode 36. Je nach seinem Schaltzustand schaltet der zweite Multiplexer 28 von der Regeleinheit 30 beispielsweise in Form von HF-Wechselspannung empfangene elektrische Leistung auf eine der Ablationselektroden 34.

Der Schaltzustand des ersten Multiplexers 26 und des zweiten Multiplexers 28 wird dabei von dem ersten Taktgeber 18 bzw. dem zweiten Taktgeber 20 bestimmt.

Die Regeleinheit 30 ist eingangsseitig über eine elektrische Leitung mit der Energiequelle 22 verbunden. Die Energiequelle 22 ist beispielsweise ein HF-Generator, der eine elektrische Wechselspannung mit einer Frequenz von 470 kHz abgibt. Die Regeleinheit 30 ist ausgangsseitig über eine elektrische Leitung mit dem zweiten Multiplexer 28 verbunden. Die Regeleinheit 30 schließt einen Verstärker ein und ist so gestaltet, daß sie die von der Energiequelle 22 empfangene Leistung geregelt und durch ein von dem dritten Taktgeber 24 empfangenes Taktsignal sowie durch ein von der Steuerungseinheit 16 empfangenes Steuerungssignal gesteuert abgibt. Dazu ist die Regeleinheit 30 eingangsseitig über eine digitale Signalleitung mit der Steuerungseinheit 16 verbunden und über eine analoge Signalleitung mit dem dritten Taktgeber 24. Die Regeleinheit 30 ist weiterhin so ausgelegt, daß die über den zweiten Multiplexer 28 an zwei Ablationselektroden 34 im bipolaren Betrieb abgegebene Leistung eine andere sein kann, als die an eine Ablationselektrode und die Neutralelektrode 36 abgegebene Leistung im unipolaren Betrieb. Die von der Regeleinheit 30 an die Elektroden 34 und 36 abgegebene Leistung, im folgenden Ablationsleistung genannt, kann außerdem in Abhängigkeit des von dem ersten Multiplexers 26 empfangenen Temperaturwertes geregelt werden.

Die Ablationselektroden 34 sind beispielsweise die Tip-Elektrode und mehrere Ringelektroden üblicher Ablationskatheter. In einen solchen Ablationskatheter sind die Temperatursensoren 32 integriert und jeweils einer der Ablationselektroden 34 des Ablationskatheters zugeordnet. Die Steuereinheit 16 steuert das Zusammenwirken der nachfolgenden Bestandteile der Ablationsanordnung 10 so, daß zum Erzeugen einer linearen Ablation zunächst die erste der Ablationselektroden (die Tip-Elektrode eines an sich bekannten Ablationskatheters) sowie die Neutralelektrode 36 mit der Hochfrequenz-Wechselspannung aus der Energiequelle 22 versorgt werden. Dabei baut sich im zu behandelnden Körper ein elektrisches Feld zwischen der großflächigen Neutralelektrode 36 und der ersten Ablationselektrode 34 auf. Die Ablationselektrode 34 besitzt wesentlich kleinere Ausmaße als die Neutralelektrode 36, mit der Folge, daß sich das elektrische Feld im Bereich der Ablationselektrode 34 bündelt. Diese Bündelung des elektrischen Feldes führt zu einer Energiedichte, die ausreicht, im Bereich der Ablationselektrode 34 befindliches Körpergewebe im gewünschten Maße zu lädieren. Anschließend werden die erste und die zweite der Ablationselektroden 34 im bipolaren Betrieb mit hochfrequenter elektrischer Energie aus der Energiequelle 22 versorgt. Dies führt zu einer Läsion zwischen der ersten und der zweiten der Ablationselektroden 34. Daraufhin wird die zweite der Ablationselektroden 34 unipolar angesteuert, d. h., die zweite der Ablationselektroden 34 und die Neutralelektrode 36 werden mit elektrischer Energie aus der Energiequelle 22 versorgt, so daß es zu einer Läsion im Bereich der zweiten der Ablationselektroden 34 kommt. Anschließend werden die zweite und die dritte der Ablationselektroden 34 wieder im Bipolarbetrieb mit hochfrequenter elektrischer Energie aus der Energiequelle 22 versorgt. Daraufhin wird die dritte der Ablationselektroden wieder unipolar, d. h. zusammen mit der Neutralelektrode 36 betrieben usw., bis die Länge der so erzielten Läsion ausreicht oder sämtliche Ablationselektroden 34 angesteuert wurden. Eine derartige Sequenz kann so oft wiederholt werden, bis die Stärke der Läsionen ausreichen. Zwischen der sequenziellen, unipolaren Abgabe von hochfrequenter Wechselspannung an die Ablationselektroden werden benachbarte Ablationselektroden kurzzeitig bipolar angesteuert. Der Ablationskatheter wird also alternierend uni- und biplar betrieben. Die Abgabe elektrischer Energie bei der bipolaren Ansteuerung kann gegenüber der unipolaren Ansteuerung reduziert oder erhöht sein, um die Tiefe der Läsionen zu steuern.

Bevor also nach einer Abgabe von Ablationsenergie in Form von hochfrequenter Wechselspannung über eine der Ablationselektroden 34 die benachbarte Ablationselektrode unipolar betrieben wird, wird über diese beiden benachbarten der Ablationselektroden 34 Hochfrequenzenergie im bipolaren Modus abgegeben. Die abgegebene Ablationsenergie kann dabei jeweils in Abhängigkeit der Ablationselektroden temperaturgeregelt werden. Dazu sind die Temperatursensoren 32, wie beschrieben, jeweils in der Nähe einer der Ablationselektroden 34 angeordnet. Die Messung der jeweiligen Ablationselektrodentemperatur erfolgt durch den zugeordneten der Temperatursensoren 32 jeweils synchron zur Energieabgabe über die jeweilige der Ablationselektroden 34. Diese Synchronizität wird durch entsprechende Ansteuerung der ersten und zweiten Multiplexer 26 und 28 erzielt. Der entsprechende Temperaturwert wird der Regeleinheit 30 zugeführt, während diese die über die Ablationselektroden bzw. über eine Ablationselektrode und die Neutralelektrode 36 abgegebene Ablationsleistung regelt. Die Regeleinheit 30 ist dabei so ausgelegt, daß Unterschiede zwischen den gemessenen Temperaturen beim bipolaren und beim unipolaren Betrieb bei der Regelung der Ablationsleistung berücksichtigt werden.

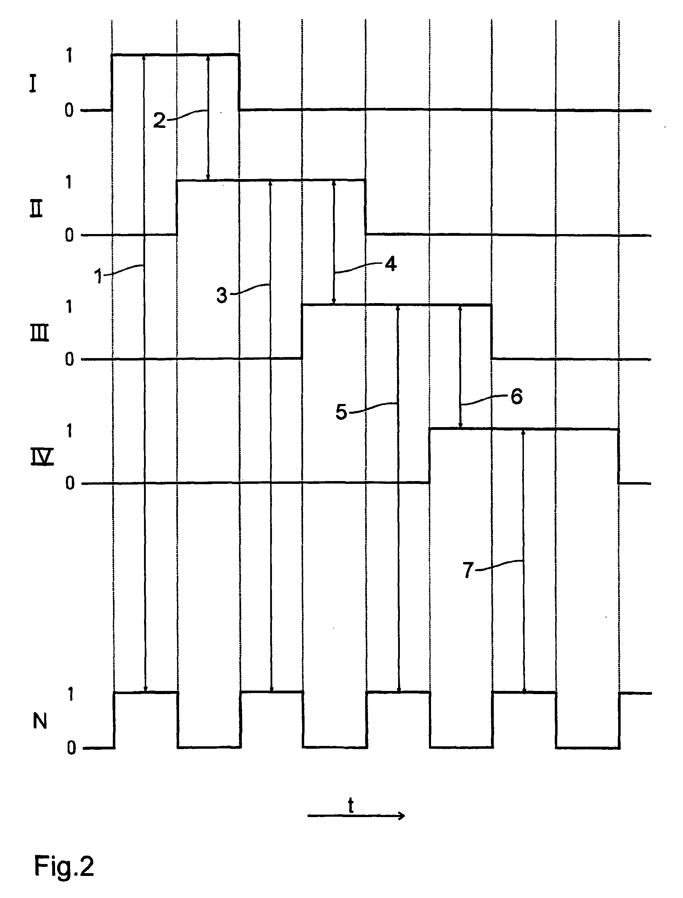

Das in Fig. 2 abgebildete Impulsdiagramm zeigt, wie die einzelnen Ablationselektroden 34 und die Neutralelektrode 36 für den beschriebenen, streng abwechselnden unipolaren und bipolaren Betrieb angesteuert werden. Im mit "1" bezeichneten Zustand ist die jeweilige Elektrode mit der Energiequelle 22 verbunden, während sie in dem "0" bezeichneten Zustand von der Energiequelle 22 getrennt ist. Die mit I. bezeichnete Impulsfolge stellt dabei den Ansteuerungszustand der ersten der Ablationselektroden 34 dar. Dies wird in der Regel die Tip-Elektrode eines Ablationskatheters sein. Die mit 11. bezeichnete Impulsfolge stellt den Ansteuerungszustand der nächstfolgenden, zweiten Ablationselektrode dar, usw., während die mit "N" bezeichnete Impulsfolge den Ansteuerungszustand der Neutralelektrode darstellt. Der Wechsel zwischen unipolaren und bipolaren Betrieb ist durch gepunktete vertikale Linien dargestellt. Der Takt, in dem dieser Wechsel stattfindet, wird durch die von der Steuerungseinheit 16 angesteuerten Taktgeber 18, 20 und 24 bestimmt. Die in Fig. 2 abgebildeten Impulsfolgen sind über die von links nach rechts fortschreitende Zeit aufgetragen. Die in Fig. 2 eingetragenen Pfeile 1 bis 7 bezeichnen die jeweils zusammen wirkenden Elektroden, nämlich Pfeil 1 das Zusammenwirken der ersten Ablationselektrode mit der Neutralelektrode im unipolaren Betrieb, Pfeil 2 das Zusammenwirken der ersten und der zweiten Ablationselektrode im bipolaren Betrieb, Pfeil 3 das Zusammenwirken der zweiten Ablationselektrode und der Neutralelektrode im unipolaren Betrieb, Pfeil 4 das Zusammenwirken der zweiten und der dritten Ablationselektrode im bipolaren Betrieb, Pfeil 5 das Zusammenwirken der dritten Ablationselektroden und der Neutralelektrode im unipolaren Betrieb, Pfeil 6 das Zusammenwirken der dritten und der vierten Ablationselektrode im bipolaren Betrieb und Pfeil 7 das Zusammenwirken der vierten Ablationselektrode und der Neutralelektrode im unipolaren Betrieb.

Die Sequenz, in der die Elektroden angesteuert werden, kann beliebig oft wiederholt werden, bis ein gewünschtes Ablationsergebnis erzielt ist. Außerdem können einige Elektroden in der Sequenz ausgelassen werden, um abschnittsweise unterbrochene Läsionen zu erzeugen.

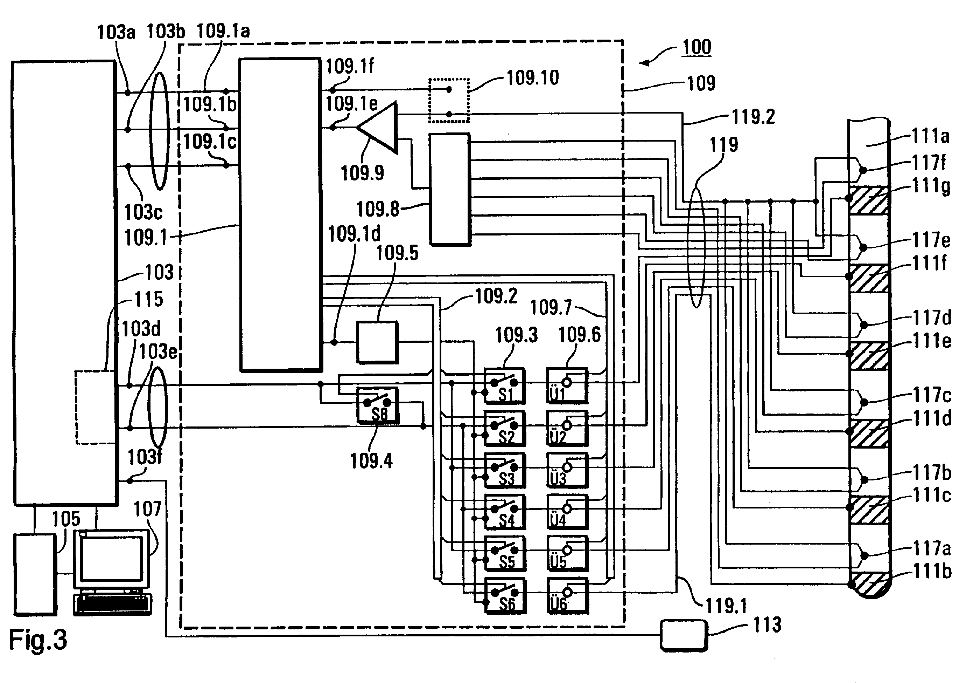

Fig. 3 zeigt eine in Teilen alternative Ausführungsform der Ablationsanordnung in einem Blockschaltbild. Die in Fig. 3 dargestellte Ablationsanordnung 100 besteht aus den Komponenten Ablationsgerät 103, Stimulationsgerät 105, klinischer Meßplatz 107, Schaltgerät 109, multipolarer Ablationskatheter 111 und Flächenelektrode 113, welche in bekannter Weise zusammenwirken. Für die Erläuterung der Erfindung sind dabei das Ablationsgerät 103, der HF-Generator 115 und die Ausgangsanschlüsse von besonderem Interesse. Dies sind zwei serielle Datenanschlüsse 103a, 103b, ein Stromversorgungsanschluß 103c, ein HF-Leistungsausgang 103d, 103e und ein Referenzanschluß 103f für die Flächenelektrode 113.

Auch der Ablationskatheter 111 ist von an sich bekannter Bauart und weist auf einem isolierenden, aus einem biokompatiblen Kunststoff gefertigten Katheterkörper 111a neben einer Tip- oder Spitzenelektrode 111 b fünf äquidistant zueinander bzw. zur Spitzenelektrode angeordnete Ringelektroden 111c bis 111 g auf, die - ebenso wie die Spitzenelektrode 111a - vorzugsweise aus Platin oder einer Pt-Ir-Legierung gefertigt sind und zusammen mit der Spitzenelektrode die Ablationselektroden bilden. Der Katheterkörper 111 a nimmt (nicht einzeln bezeichnete) Zuleitungen für die einzelnen Elektroden sowie die - ebenfalls nicht einzeln bezeichneten - Konstantan- bzw. Kupferdrähte für den Elektroden räumlich zugeordnete Thermoelemente 117a bis 117f auf. In einer Verbindungsleitung 119 sind HF-Leitungen 119.1 und die Temperatursignalleitungen 119.2 vom Schaltgerät 119 zum Ablationskatheter 111 geführt.

Das Schaltgerät ist eingangsseitig (über wiederum nicht gesondert bezeichnete Leitungen) mit den Ausgangsanschlüssen 115a bis 115e des Ablationsgerätes verbunden. Es umfaßt als wichtigste Komponenten für die Ablationssteuerung einen Mikrocontroller 109.1, sechs mit dem Mikrocontroller über eine Busleitung 109.2 verbundene elektronische Schalter 109.3 für die Katheterelektroden 111b bis 111g sowie einen Sicherheitsausschalter 109.4 und schließlich einen Watchdog-Timer 109.5. Den HF-Ausgangsleitungen 119.1 sind Stromüberwachungsfühler 109.6 zugeordnet, die über eine zweite Busleitung 109.7 mit dem Mikrocontroller 109.1 verbunden sind.

Der Mikrocontroller ist über Datenein- und -ausgänge 109.1 a. 109.1 b mit den Datenein- und -ausgängen 103a, 103b des Ablationsgerätes und über einen Stromversorgungs-Eingang 109.1 c mit dessen Stromversorgungs-Ausgang 103c verbunden, und für den Watchdog-Timer 109.5 ist ein separater Steuerausgang 109.1 d vorgesehen.

Die Cu-Leitungen der Thermoelemente 117a bis 117f sind über einen Multiplexer 109.8 und die Konstantan-Leitung direkt mit einem einzelnen Meßverstärker 109.9 verbunden, und dessen Ausgang ist mit einem ersten T-Signaleingang 109.1 d des Mikrocontrollers verbunden. Eine als Referenz dienende Kaltlötstelle 109.10 mit (nicht dargestelltem) Thermistor zur T-Erfassung ist direkt mit einem zweiten T-Signaleingang 109.1 e des Mikrocontrollers verbunden.

Die primären Steuerfunktionen für eine Ablationsbehandlung (Elektrodenauswahl und -ansteuerfolge, Dauer und Abstände der HF-Impulse etc.) werden nach Auswertung der mit Hilfe des Meßplatzes 107 gewonnenen Ergebnisse der klinischen Untersuchung aufgrund einer entsprechenden Programmierung durch den Arzt in dem (in der Praxis auch als "Ablator" oder "Ablationssystem" bezeichneten) Ablationsgerät 103 ausgeführt. Das Ablationsgerät 103 ist so ausgeführt, daß die Ablationselektroden innerhalb einer Sequenz sowohl unipolar als auch bipolar betrieben werden, und daß die Ablationsleistung für den unipolaren Betrieb eine andere ist, als für den bipolaren Betrieb. Steuerbefehle des Ablationsgerätes werden über die Datenausgänge 103a, 103 dem Mikrocontroller 109.1 zugeführt, der unter anderem die Steuerbefehle in Steuersignale für die Schalter 109.3 und den Multiplexer 109.9 umsetzt, die Schaltfunktionen mittels der Stromfühler 109.6 überwacht und die Temperatur an den einzelnen Elektroden 117a bis 117f berechnet.

Mit der dargestellten Schalteranordnung ist die Abgabe von HF-Energie mit jeder beliebigen Kombination von Ablationselektroden untereinander oder mit der Flächen- bzw. Neutralelektrode 113 sowie die Applikation von Stimulationsimpulsen und die Detektion des intrakardialen Elektrogramms IEGM an den jeweils zugeschalteten Elektroden möglich. Durch synchrone Steuerung des Multiplexers 109.8 kann zugleich die Temperatur an den Elektroden überwacht werden, der Multiplexer kann aber im Bedarfsfall auch asynchron zu den Schaltern gesteuert werden. Die Ergebnisse der T-Überwachung werden an das Ablationsgerät 103 zur Anzeige für den Arzt und zur etwaigen Modifikation des Ablationsprogramms übergeben, können aber auch intern im Mikrocontroller 109.1 zur Auslösung einer Elektrodenabschaltung bei unzulässig hohen T-Werten genutzt werden.

Die Schalter 109.3 und 109.4 werden bevorzugt als MOSFET-Schalter ausgeführt, können aber - sofern die prinzipbedingte Limitierung der Umschaltgeschwindigkeit hinnehmbar ist - insbesondere auch durch Relais gebildet sein. Die bidirektionale Datenübertragung zwischen Ablator 103 und Schaltgerät 109 oder eine entsprechende Programmierung des Ablators ermöglicht die kurzzeitige Austastung der HF bei jeder Elektrodenumschaltung zur Verringerung der Schalterbeanspruchung, was vor allem bei Einsatz von Relais vorteilhaft ist.

## Patentansprüche

1. Verfahren zum Ansteuern der Elektroden (34, 36) einer Ablationsanordnung (10), die einen Ablationskatheter mit einer Mehrzahl von Ablationselektroden (34) und eine Neutralelektrode (36) sowie mindestens eine Energiequelle (22) und Schaltmittel (26, 28) zum Verbinden der Elektroden (34, 36) mit der Energiequelle (22) umfaßt, wobei sowohl von den Ablatoinselektroden (34) als auch von einer Ablationselektrode (34) zusammen mit der Neutralelektrode (36) gebildete Elektrodenpaare mit der Energiequelle (22) in einer vorgegebenen Reihenfolge verbunden werden, **dadurch gekennzeichnet, daß** streng abwechselnd ein von Ablationselektroden (34) gebildetes Elektrodenpaar und ein von einer der Ablationselektroden (34) zusammen mit der Neutralelektrode (36) gebildetes Elektrodenpaar mit der Energiequelle (22) verbunden werden.

2. Verfahren nach Anspruch 1, **dadurch gekennzeichnet, daß** die von Ablationselektroden (34) gebildeten Elektrodenpaare von einander benachbarten Ablationselektroden (34) einer Mehrzahl von mit Abstand aufeinander folgenden Ablationselektroden (34) gebildet werden.

3. Verfahren nach einem der Ansprüche 1 bis 2, **dadurch gekennzeichnet, daß** zunächst ein von einer ersten einer Reihe von mit Abstand aufeinander folgenden Ablationselektroden (34) zusammen mit der Neutralelektrode (36) gebildetes Elektrodenpaar mit der Energiequelle (22) verbunden wird, anschließend ein von der ersten Ablationselektrode und einer auf diese folgenden zweiten Ablationselektrode gebildetes Elektrodenpaar, daraufhin ein von der zweiten Ablationselektrode und der Neutralelektrode (36) gebildetes Elektrodenpaar, daraufhin wieder ein von der zweiten Ablationselektrode und der nächstfolgenden Ablationselektrode gebildetes Elektrodenpaar und in dieser abwechselnden Reihe fortgefahren wird, bis der Abstand zwischen der ersten mit der Energiequelle verbundenen Elektrode und der letzten mit der Energiequelle verbundenen Elektrode einer gewünschten Länge entspricht.

4. Verfahren nach einem der Ansprüche 1 bis 3, **dadurch gekennzeichnet, daß** eine oder mehrere Ablationselektroden (34) in der Reihenfolge, der mit der Energiequelle (22) zu verbindenden Elektroden ausgelassen werden, um Ablationslücken zu lassen.

5. Verfahren nach einem der Ansprüche 1 bis 4, **dadurch gekennzeichnet, daß** die Leistung, mit der die von der Energiequelle (22) stammende Energie über die Elektroden (34, 36) abgegeben wird, eine andere ist, wenn ein Elektrodenpaar von zwei Ablationselektroden (34) gebildet wird, als wenn ein Elektrodenpaar von einer Ablationselektrode (34) zusammen mit der Neutralelektrode (36) gebildet wird.

6. Verfahren nach einem der Ansprüche 1 bis 5, **dadurch gekennzeichnet, daß** die Leistung, mit der die von der Energiequelle (22) stammende Energie über zwei Ablationselektroden (34) abgegeben wird, derart durch Messen der Elektrodentemperatur geregelt wird, daß für die Regelung zwei Temperaturwerte berücksichtigt werden, die jeweils der einen und der anderen Ablationselektrode (34) zugeordnet sind.

7. Ablationsanordnung, umfassend einen Ablationskatheter mit einer Mehrzahl von Ablationselektroden (34) und eine Neutralelektrode (36) sowie mindestens eine Energiequelle (22) und Schaltmittel (26, 30) zum Verbinden der Elektroden mit der Energiequelle (22), wobei die Schaltmittel (26, 30) mit Steuermitteln (16, 18, 20, 24), verbunden sind, die derart gestaltet sind, daß sowohl von den Ablationselektroden (34) als auch von einer Ablationselektrode zusammen mit der Neutralelektrode (36) gebildete Elektrodenpaare mit der Energiequelle (22) in einer vorgebbaren Reihenfolge verbindbar sind, **dadurch gekennzeichnet, daß** die Steuermittel (16, 18, 20, 24) derart gestaltet sind, daß sie streng abwechselnd ein von Ablationselektroden gebildetes Elektrodenpaar und ein von einer der Ablationselektroden zusammen mit der Neutralelektrode gebildetes Elektrodenpaar mit der Energiequelle (22) verbinden.

8. Ablationsanordnung nach Anspruch 7, **dadurch gekennzeichnet, daß** die Ablationselektroden (34) mit Abstand aufeinander folgend angeordnet sind und die Steuermittel (16, 18, 20, 24) derart gestaltet sind, daß die von Ablationselektroden (34) gebildeten Elektrodenpaare von einander benachbarten Ablationselektroden gebildet sind.

## Claims

1. A method for activating the electrodes (34, 36) of an ablation configuration (10), which comprises an ablation catheter having multiple ablation electrodes (34) and a neutral electrode (36), as well as at least one energy source (22) and switching means (26, 28) for connecting the electrodes (34, 36) to the energy source (22), electrode pairs both formed by the ablation electrodes (34) and also formed by one ablation electrode (34) together with a neutral electrode (36) being connected to the energy source (22) in a predefined sequence, **characterized in that** an electrode pair formed by ablation electrodes (34) and an electrode pair formed by one of the ablation electrodes (34) together with a neutral electrode (36) are connected to the energy source (22) in strict alternation.

2. The method according to Claim 1, **characterized in that** the electrode pairs formed by ablation electrodes (34) are formed by neighboring ablation electrodes (34) of multiple ablation electrodes (34) following one another at a distance.

3. The method according to one of Claims 1 through 2, **characterized in that** firstly an electrode pair formed by one of a first series of ablation electrodes (34), which follow one another at a distance, together with the neutral electrode (36) is connected to the energy source (22), subsequently an electrode pair formed by the first ablation electrode and a second ablation electrode following this, then an electrode pair formed by the second ablation electrode and the neutral electrode (36), subsequently again an electrode pair formed by the second ablation electrode and the next following ablation electrode, and this alternating series being continued until the distance between the first electrode connected to the energy source and the last electrode connected to the energy source corresponds to a desired length.

4. The method according to one of Claims 1 through 3, **characterized in that** one or more ablation electrodes (34) in the sequence of the electrodes to be connected to the energy source (22) are left out to leave ablation gaps.

5. The method according to one of Claims 1 through 4, **characterized in that** the power at which the energy originating from the energy source (22) is delivered via the electrodes (34, 36) is different when an electrode pair is formed by two ablation electrodes (34) than when an electrode pair is formed by an ablation electrode (34) together with a neutral electrode (36).

6. The method according to one of Claims 1 to 5, **characterized in that** the power at which the energy originating from the energy source (22) is delivered via two ablation electrodes (34) is regulated by measuring the electrode temperature in such a way that two temperature values are considered for the regulation, one of which is assigned to one ablation electrode (34) and the other of which is assigned to the other ablation electrode.

7. An ablation configuration, comprising an ablation catheter having multiple ablation electrodes (34) and a neutral electrode (36), as well as at least one energy source (22) and switching means (26, 30) for connecting the electrodes to the energy source (22), the switching means (26, 30) being connected to control means (16, 18, 20, 24), which are designed in such a way that both electrode pairs formed by the ablation electrodes (34) and also by an ablation electrode together with a neutral electrode (36) are connectable to the energy source (22) in a predefinable sequence, **characterized in that** the control means (16, 18, 20, 24) are designed in such a way that they connect an electrode pair formed by ablation electrodes and an electrode pair formed by one of the ablation electrodes together with the neutral electrode to the energy source (22) in strict alternation.

8. The ablation configuration according to Claim 7, **characterized in that** the ablation electrodes (34) are situated one after another at a distance and the control means (16, 18, 20, 24) are designed in such a way that the electrode pairs formed by ablation electrodes (34) are formed by neighboring ablation electrodes.

## Revendications

1. Procédé pour l'activation des électrodes (34, 36) d'un dispositif d'ablation (10), qui comprend un cathéter d'ablation avec une pluralité d'électrodes d'ablation (34) et une électrode neutre (36) ainsi qu'au moins une source d'énergie (22) et des moyens de commutation (26, 28) pour la liaison des électrodes (34, 36) avec la source d'énergie (22), des paires d'électrodes formées aussi bien par les électrodes d'ablation (34) que par une électrode d'ablation (34) conjointement avec l'électrode neutre (36) étant reliées à la source d'énergie (22) dans un ordre de succession prédéfini, **caractérisé en ce que**, avec une alternance stricte, une paire d'électrodes formée par des électrodes d'ablation (34) et une paire d'électrodes formée par l'une des électrodes d'ablation (34) conjointement avec l'électrode neutre (36) sont reliées à la source d'énergie (22).

2. Procédé selon la revendication 1, **caractérisé en ce que** les paires d'électrodes formées par des électrodes d'ablation (34) sont formées par des électrodes d'ablation (34) voisines entre elles d'une pluralité d'électrodes d'ablation (34) se suivant à distance.

3. Procédé selon l'une quelconque des revendications 1 à 2, **caractérisé en ce que** d'abord une paire d'électrodes formée par une première d'une série d'électrodes d'ablation (34) se succédant à distance conjointement avec l'électrode neutre (36) est reliée à la source d'énergie (22), ensuite une paire d'électrodes formée par la première électrode et une seconde électrode d'ablation suivant la première, ensuite une paire d'électrodes formée par la seconde électrode d'ablation et l'électrode neutre (36), ensuite à nouveau une paire d'électrodes formée par la seconde électrode d'ablation et l'électrode d'ablation suivante, et on poursuit dans cet ordre d'alternance jusqu'à ce que la distance entre la première électrode reliée à la source d'énergie et la dernière électrode reliée à la source d'énergie corresponde à une longueur souhaitée.

4. Procédé selon l'une quelconque des revendications 1 à 3, **caractérisé en ce que** une ou plusieurs électrodes d'ablation (34) sont laissées éteintes dans l'ordre de succession des électrodes à relier à la source d'énergie, afin de laisser des vides d'ablation.

5. Procédé selon l'une quelconque des revendications 1 à 4, **caractérisé en ce que** la puissance, avec laquelle l'énergie provenant de la source d'énergie (22) est délivrée sur les électrodes (34, 36), est une autre électrode lorsqu'une paire d'électrodes est formée par deux électrodes d'ablation (34) et lorsqu'une paire d'électrodes est formée par une électrode d'ablation (34) conjointement avec l'électrode neutre (36).

6. Procédé selon l'une quelconque des revendications 1 à 5, **caractérisé en ce que** la puissance, avec laquelle l'énergie provenant de la source d'énergie (22) est délivrée sur les deux électrodes d'ablation (34), est réglée par la mesure de la température d'électrodes de telle sorte que, pour le réglage, on prend en compte deux valeurs de températures qui sont attribuées chacune à l'une et à l'autre des électrodes d'ablation (34).

7. Agencement d'ablation comprenant un cathéter d'ablation avec une pluralité d'électrodes d'ablation (34) et une électrode neutre (36) ainsi qu'au moins une source d'énergie (22) et des moyens de commutation (26, 30) pour la liaison des électrodes avec la source d'énergie (22), les moyens de commutation (26, 30) étant reliés à des moyens de commande (16, 18, 20, 24) qui sont conçus de telle sorte que des paires d'électrodes formées aussi bien par les électrodes d'ablation (34) que par une électrode d'ablation conjointement avec l'électrode neutre (36) peuvent être reliées à la source d'énergie (22) dans un ordre de succession prédéfinissable, **caractérisé en ce que** les moyens de commande (16, 18, 20, 24) sont conçus de telle sorte qu'ils relient avec une alternance stricte une paire d'électrodes formée par des électrodes d'ablation et une paire d'électrodes formée par l'une des électrodes d'ablation conjointement avec l'électrode neutre à la source d'énergie (22).

8. Agencement d'ablation selon la revendication 7, **caractérisé en ce que** les électrodes d'ablation (34) sont disposées à distance les unes des autres et en se suivant et les moyens de commande (16, 18, 20, 24) sont conçus de telle sorte que les paires d'électrodes formées par des électrodes d'ablation (34) sont formées par des électrodes d'ablation respectivement voisines les unes des autres.
